# EUROPEAN PATENT SPECIFICATION

(11) **EP 2 152 259 B1**
(45) Date of publication and mention of the grant of the patent: **31.07.2013**
(21) Application number: 08750041.9
(22) Date of filing: 05.05.2008
(51) Int. Cl.: A61K 31/192, A61K 41/00, A61K 45/06, A61P 17/10, A61N 5/06

(54) **USE OF A NAPHTOIC ACID DERIVATIVE IN COMBINATION WITH RED AND/OR BLUE LIGHT FOR TREATING ACNE VULGARIS**
VERWENDUNG EINES NAPHTOESÄUREDERIVATS IN KOMBINATION MIT ROTEM UND/ODER BLAUEM LICHT ZUR BEHANDLUNG VON AKNE VULGARIS
UTILISATION D'UN DÉRIVÉ D'ACIDE NAPHTOÏQUE COMBINÉE À UNE LUMIÈRE ROUGE ET/OU BLEUE DANS LE TRAITEMENT D'ACNÉ VULGAIRE

(30) Priority: 04.05.2007 US 924253 P
(43) Date of publication of application: 17.02.2010
(73) Proprietor: Galderma Research & Development, 06410 Biot (FR)
(72) Inventor: COLON, Lucy, Fort Worth, Texas 76177 (US)
(74) Representative: Macquet, Christophe
(86) International application number: PCT/EP2008/055481
(87) International publication number: WO 2008/135548

(56) References cited:
- WO-A-03/086215
- WO-A-2006/012752
- WO-A-2007/103132
- NESTOR M S: "The Use of Photodynamic Therapy for Treatment of Acne Vulgaris" DERMATOLOGIC CLINICS - PHOTODYNAMIC THERAPY 200701 US, vol. 25, no. 1, January 2007 (2007-01), pages 47-57, XP009106128 ISSN: 0733-8635

## Description

This invention relates to a method of treatment of acne vulgaris with naphtoic acid derivative and particularly adapalene in association/combination with light therapy.

Acne vulgaris is a common skin disorder that makes up 20% of the visits to a dermatology practice, and affects the majority of the teenage population. Management of acne is challenging, especially when considering the chronicity of the disease and the variability in response to treatment.

The recent Consensus Recommendations for the Management of Acne (JAAD sup 2003; 49:1), state that effective acne treatment should target as many of its pathogenic factors, as possible.

The recommendations also state that a topical retinoid should be used in the initial treatment of almost all new patients with acne, because they are the most effective anticomedonal agents currently available. Retinoids help disrupt acne pathogenesis by preventing the development of new microcomedones, and some possess both direct and indirect anti-inflammatory activity. Because retinoids do not possess anti-bacterial activity, the use of another agent may also be necessary to treat inflammatory activity. Retinoids enhance the follicular penetration of other agents and thus help in overall effectiveness.

The management of acne often requires combination therapy and a long-term therapeutic strategy. (See, for example, Thiboutot D. New treatments and therapeutic strategies for acne. Arch Family Med 2000; 9: 179-187; Gollnick H, Cunliffe W, Berson D, et al. Management of acne, a report from a Global Alliance to Improve Outcomes in Acne. J Am Acad Dermatol. 2003;49(1 suppl):S1-S37).

Exposure to sunlight has been known to improve acne. Newer acne therapy that combines narrow bands of blue and red lights (415 and 660 nm)-without other treatment agent- has been considered effective because it uses antibacterial and anti-inflammatory mechanisms, with no side-effects. This therapy using visible light is based on light absorption by *P acnes,* which have porphyrins that absorb maximally at the blue light wavelength of 415 nm. Blue light is considered useful because its intensity can excite the porphyrins and lead to the production of oxygen free radicals and phototoxic effects, therefore reducing acne lesions. The porphyrins can also absorb the longer red light wavelengths, which penetrate deeper into the skin. In addition, red light is known to have anti-inflammatory properties, influencing macrophages to release cytokines that stimulate the release of growth factors, which are known to influence the process of inflammation, healing and wound repair (Fien, S, Ballard C, Nouri K. Multiple modalities to treat acne: A review of lights, lasers and radiofrequency. Cosmetic Dermatology (December 2004);17(12):789-793)).

WO 2006/012752 discloses a therapy device using electromagnetic radiation which can be used for the treatment of acne.

The physical modality of this therapy, has been found to be comparable to treatment with topical clindamycin but inferior to benzoyl peroxide plus clindamycin (Abramovits, W, Arrazola, P, Gupta A. Light-emitting diode-based therapy. SKINmed.(January-February 2005);4(1): 38-41).

Although recommendation state that a topical retinoid should be used in the initial treatment of almost all new patients with acne, there is no report on the use of retinoids with visible light treatment.

However, treated skin exposure to sunlight by any retinoid treatment is not recommended as it might induce photosensitivity reactions.

The present invention provides a new method of treatment of acne related diseases and particularly acne vulgaris, said method comprising administering to a patient in need a therapeutical effective amount of a retinoid and particularly naphthoic acid derivative in combination/association with light exposure. The naphtoic acid is adapalene.

The present invention provides a regime or a regimen for inhibiting or treating acne related diseases and in particular acne vulgaris, comprising administering to an individual subject in need of treatment an effective amount of naphtoic acid derivative which is adapalene in association or combination with red and/or blue light exposure. In a preferred embodiment, the said acne related disease is acne vulgaris. According to this regimen, the treatment with adapalene and light exposure is carried out at least once per day between every two day to once a week and in a preferred embodiment once/day twice a week. According to a particular embodiment of the regime or a regimen, the Adapalene treated afflicted skin is exposed to blue light first and red light then. Preferably, the Adapalane treated afflicted skin is exposed to blue light the first week and the red light exposure is carried out the weeks following. In the context of the invention, the blue light is emitted with length waves comprised between 405 nm and 420 nm, preferably between 410 nm and 420 nm, most preferred at 415 nm; and the red light is emitted with length waves comprised between 620 nm and 650 nm, preferably between 630 nm and 640 nm, most preferred at 633 nm.

The regime or the regimen is carried out for at least 3 weeks and preferably over a period between 4 and 12 weeks. The treatment with Adapalene and light exposure is carried out on skin, preferably on face, trunk and back.

In another embodiment, the present invention provides a method for treating a patient afflicted with acne related disease and particularly acne vulgaris comprising administering to a patient in need of treatment, an effective amount of adapalene in association or combination with red and/or blue light exposure.

In a preferred embodiment, the said acne related disease is acne vulgaris. According to this method for treating a patient, the treatment with adapalene and light exposure is carried out at least once per day between every two day to once a week and in a preferred embodiment once/day twice a week. According to a particular embodiment of the method for treating a patient, the Adapalene treated afflicted skin is exposed to blue light first and red light then. Preferably, the Adapalene treated afflicted skin is exposed to blue light the first week and the red light exposure is carried out the weeks following. In the context of the invention, the blue light is emitted with length waves comprised between 405 nm and 420 nm, preferably between 410 nm and 420 nm, most preferred at 415 nm; and the red light is emitted with length waves comprised between 620 nm and 650 nm, preferably between 630 nm and 640 nm, most preferred at 633 nm.

The method for treating a patient is carried out for at least 3 weeks and preferably over a period between 4 and 12 weeks. The treatment with adapalene and light exposure is carried out on skin, preferably on face, trunk and back.

In another embodiment, the present invention relates to the use of a naphtoic acid derivative which is Adapalene in the preparation of a topical medicament for administering to a patient in need so as to provide a treatment of acne vulgaris in association or combination with red and/or blue light.

In a preferred embodiment, the said acne related disease is acne vulgaris. According to this use of preparation of topical medicament, the treatment with Adapalene and light exposure is carried out at least once per day between every two day to once a week and in a preferred embodiment once/day twice a week. According to a particular embodiment of use of preparation of topical medicament, the Adapalene treated afflicted skin is exposed to blue light first and red light then. Preferably, the Adapalene treated afflicted skin is exposed to blue light the first week and the red light exposure is carried out the weeks following. In the context of the invention, the blue light is emitted with length waves comprised between 405 nm and 420 nm, preferably between 410 nm and 420 nm, most preferred at 415 nm; and the red light is emitted with length waves comprised between 620 nm and 650 nm, preferably between 630 nm and 640 nm, most preferred at 633 nm.

The use of preparation of topical medicament is carried out for at least 3 weeks and preferably over a period between 4 and 12 weeks. The treatment with Adapalene and light exposure is carried out on skin, preferably on face, trunk and back.

Preferably, the topical medicament is an aqueous gel composition. More particularly, the naphtoic acid derivative is adapalene at a concentration of 0,1% in weight with regards to the total weight of the composition. In a specifically preferred embodiment adapalene is adapalene 0,1% Gel.

In the context of the present invention, the term "naphtoic acid derivative"

means adapalene and its salts. It is meant by adapalene salts, the salts obtained or obtainable with a pharmaceutical acceptable base, particularly mineral bases such as sodium hydroxide, potassium hydroxide, ammonium hydroxyde or organic bases such as lysine, arginine, N-methyl-glucamine. It is also meant the salts obtained or obtainable with fatty amine such as dioctylamine and stearylamine.

The abovementioned adapalen is generally in a form dispersed in the composition according to the invention.

In the compositions according to the invention, Adapalene is used at concentrations of less than or equal to 10% by weight relative to the total weight of the composition, and preferably between 0.001% and 10% by weight relative to the total weight of the composition, preferentially between 0.01% and 5%, more preferentially between 0.05% and 2% and most preferentially from 0.1 % to 0.3% by weight relative to the total weight of the composition. Throughout the present text, unless otherwise specified, it is understood that when ranges of concentrations are given, the upper and lower limits of the said range are included.

The naphthoic acid derivative used in the compositions according to the invention is 6-(3-(1-adamantyl)-4-methoxyphenyl]-2-naphthoic acid also named adapalene. The adapalene concentration used in the composition according to the invention is then between 0.001 and 5% and advantageously between 0.01% and 1 % in weight of adapalene with regards to the total weight of the composition, preferably between 0.01% and 0.5%, and preferentially at least equal to 0.03%, more preferentially between 0.1 % and 0.4% and particularly preferred at a concentration of 0.1% and at a concentration of 0.3%.

Preferably, Adapalene is (are) the only active principle(s) present in the composition according to the invention.

Adapalene was developed for the topical treatment of acne vulgaris and other retinoid-sensitive dermatoses including various disorders of keratinisation, proliferation and differentiation. Adapalene acts mainly by regulating differentiation of keratinocytes (comedolytic effect and preventing new comedones), but also has anti-inflammatory activity.

In the context of the present invention the term acne related diseases means common acne or acne vulgaris, comedones, polymorphous acne, nodulocystic acne, acne conglobata, secondary acne such as solar, drug-related or occupational acne.

As mentioned above, an embodiment of present invention is to provide the use of adapalene in the preparation of a topical medicament for administering to a patient so as to treat acne related disease in association/combination with red and/or blue light. Particularly, the use of adapalene is to treat acne vulgaris.

As mentioned above, one object of the present invention is to provide a regime or a regimen for inhibiting or treating acne related diseases and in particular acne vulgaris, comprising administering to an individual subject in need of treatment an effective amount of naphtoic acid derivative which is adapalene in association/combination with red and/or blue light exposure.

As mentioned above, another object of the present invention is to provide a method for treating a patient afflicted with acne related disease and particularly acne vulgaris comprising administering to a patient in need of treatment, an effective amount of naphtoic acid derivative which is adapalene in association/combination with red and/or blue light exposure.

The said method consists in particular to apply topically to the afflicted skin region of the patient a topical medicament (which is here a dermatological preparation) comprising a therapeutically effective amount of naphotic acid derivative which is adapalene, at least once/day between every three day to once a week and exposing said treated afflicted skin region to red and/or blue light. In a particular embodiment, the treatment with naphotic acid derivative, which is adapalene, and light exposure is carried out once/day twice a week.

In a specific embodiment of the invention, the naphotic acid derivative, which is adapalene, treated afflicted skin is exposed to blue light first and red light then. In particular, the exposure to blue light is carried out the first week and the red light exposure is carried out the weeks following.

The light exposure treatment can be achieved by any red or blue light emitting device. For instance, the Omnilux™ system based on narrowband Light Emitting Diodes (LEDSs) is appropriate in the context of the instant invention. Omnilux™ light therapy is non-invasive, non-ablative and safe for acne patients. It stimulates cells athermally without causing damage to the epidermis or dermal tissue.

The light exposure treatment is comprised between 10 minutes and 30 minutes, and preferably between 15 to 25 minutes. Most preferred 20 minutes for blue light and 16 minutes for red light.

The topical medicament, which is a dermatological preparation, can be applied to the afflicted skin region in the evening after wash, preferably once daily. Preferably, the dermatological preparation is an aqueous gel composition comprising a higher strength, i.e., at least 0.2%.

The described method is particularly indicated for treating the back and the face of subject suffering of acne vulgaris and especially with mild to moderate acne vulgaris measured by subjects with a minimum of 15 inflammatory lesions (papules and pustules) on the face, subjects with a minimum of 30 non-inflammatory lesions (open and closed comedones) on the face.

For a better understanding of the invention, its operating advantages, and specific objects attained by its use, reference should be had to the descriptive matter in which there are illustrated and described preferred embodiments of the invention.

### EXAMPLE : Clinical test of treatment of acne vulgaris with a gel composition containing 0.1 % by weight of adapalene in association with exposure to light

This example is the first study evaluating the concomitant use of these treatments.

Differin is a commonly prescribed medication for the treatment of acne vulgaris. Differin® (adapalenc gel,) Gel, 0.1% is a clear gel that is odorless, oil-free and alcohol-free. Differin® Gel, is applied directly to the face or affected area, and is thought to work deep inside the follicles to control the cause of the buildup that leads to the formation of acne, by normalizing the improper accumulation of skin cells.

The Omnilux™ system is equipped to irradiate the porphyrins in acne associated bacteria with wavelengths of light specifically designed to stimulate these porphyrins into synthesizing intracellular singlet oxygen, which in turn induces bacterial death and so eradicates the inflammatory effects of acne. Clinical trials with the proposed light therapy have displayed optimum acne clearance when blue (415 nm) light is combined with red light (633 nm) treatment.

The study evaluates the safety and efficacy of treatment of acne using Blue and Red light in combination with Differin® Gel, 0.1 % as compared to Blue and Red light alone and Differin® Gel, 0.1 % alone.

Treatment with Differin® Gel, 0.1% alone were limited to 12 weeks as this was the length of the treatment in clinical studies supporting the prescription product. For mild to moderate acne, the manufacturer's recommendation for blue light/red light therapy is eight alternating 20 minute treatments over a 4 week period. In this study, the recommended 4 week therapy regimen for the blue light/red light will be evaluated by itself and when used concurrently with a 12-week treatment with Differin® Gel, 0.1%.

This study design was chosen to insure the most reliable data for a true comparison between the three different regimens. The design involves a parallel group comparison where the evaluator is blinded. This design is widely accepted to provide reliable comparative data.

### METHODS

### Study design and subjects

### NUMBER OF SUBJECTS

A total of 60 Subjects were enrolled (20 in the combination of Blue Light, Red Light and Differin® Gel, 0.1% group; 20 in the Blue light and Red Light alone group; and 20 in the Differin® Gel, 0.1% alone group) at 1 study site.

### STUDY POPULATION CHARACTERISTICS

Male or female Subjects of any race or ethnicity, aged 12 years old or older with a diagnosis of mild to moderate acne vulgaris.

### OVERALL STUDY DESIGN

This study was conducted as a single-center, randomized, single blind trial (evaluator blinded) involving Subjects of any race, age 12 years and older with mild to moderate acne vulgaris, and meeting other specific inclusion/exclusion criteria.

A total of 60 Subjects were enrolled (20 in the combination of Blue Light, Red Light and Differin® Gel, 0.1% group, 20 in the Blue Light and Red Light alone group, and 20 in the Differin® Gel,0.1% group) in one investigative site in the United States.

Subjects were randomized in a 1:1:1 ratio to one of three treatment groups:
- One group of Subjects received concomitant therapy with Red Light and Blue Light biweekly for 4 weeks and 12 weeks of treatment with Differin® Gel, 0.1 %.
- The second group of Subjects was randomized to therapy with Blue Light and Red Light biweekly for 4 weeks.
- In the last group, Subjects were randomized to 12 weeks of treatment with Differin® Gel, 0.1 1%. All subjects were provided with Cetaphil® Daily Facial Cleanser and instructed to use the cleanser twice daily throughout their treatment.

There were 4 study visits: baseline, week 4, week 8, and week 12 for all three treatment groups.

### STUDY PRODUCTS

### Differin® Gel, 0.1%

Differin® is a commonly prescribed medication for the treatment of acne vulgaris. Differin® (adapalene gel) Gel, 0.1% is a clear gel that is odorless, oil-free and alcohol-free.

### Omnilux™ System

The Omnilux™ system is based on narrowband Light Emitting Diodes (LEDSs). Clinicians can operate multiple treatment heads from a single operational base. The Onmilux™ treatment heads emit light at specific, individual narrowband wavelengths to ensure optimal photobiomodulation of the target skin cells and treatment in a wide range of dermatological conditions, including acne.

Omnilux™ light therapy is non-invasive, non-ablative and safe for acne patients. It stimulates cells athermally without causing damage to the epidermis or dermal tissue, therefore it is expected that there will be no downtime for subjects or unwanted side effects, such as erythema, peeling or blistering.

### Treatment Assignment

Prior to the start of the study, a randomization list was generated by a designated statistician from Galderma Laboratories L.P.

In this study 20 subjects were enrolled in the Blue Light and Red Light and Differin® Gel, 0.1 % treatment group; 20 subjects were enrolled in the Blue Light and Red Light alone group; and 20 subjects were enrolled in the Differin® Gel, 0.1% alone group. This is a single-center study located in the United States Treatment assignment was determined by a 1:1:1 randomization. The investigative staff member responsible for dispensation assigned the next treatment and/or combination as provided by the designated statistician. The evaluator was not be involved in the receipt and storage of drug products in order to protect the blinding.

For treatment allocation at baseline visit, each Subject, who fulfils all criteria to receive the study treatment, was assigned a Subject number. Treatment was assigned to the Subjects in the chronological order of their inclusion in the study, and no number has to be omitted or skipped.

Each subject received both written and verbal instructions as to the proper dosing and study product application techniques. Those subjects randomized to the groups using Differin® Gel, 0.1% were instructed to apply study product once a day, in the evening for 12 weeks. The first dose of study product was applied by the Subject under the direction of study personnel before leaving the investigational site.

All Subjects were applied study product after washing their face. Medicated shaving creams have not be used during the study. During the initial interview, subjects were queried as to whether or not they use shaving cream on their face. If they use shaving cream, the brand will be recorded on the Case Report Form.

The treatment administration is further described below:
Differin® Gel, 0.1%
Dose Regimen Once daily at night
Period of Administration 12 weeks
Route of Administration Topical application
Subjects randomized to the combination light therapy and light therapy alone received instructions as to when to return to the investigative site for blue light/red light treatment. The first study treatment (Blue Light) was administered on Day 1. The second study treatment was administered later that same week. The study treatments (Blue and Red Light) was administered twice a week (Blue Light on the first weekly visit and Red Light on the second weekly visit) for a total of 4 weeks with a total of 8 treatments.

### EFFICACY AND SAFETY ASSESSMENT

Clinical evaluations were performed by the same evaluator throughout the study. If it was not possible to use the same evaluator to follow the Subject, then evaluations should have overlapped (examine the Subject together and discuss findings) for at least one visit.

The safety variables evaluated were: local tolerability (erythema, scaling, dryness, and stinging/burning), Adverse Events (AEs), and routine laboratory data (hematology, blood chemistry, and urinalysis). Side effects expected during treatment with topical retinoids include erythema, scaling, dryness, and stinging/burning. During the study, the course of these expected events was assessed as local tolerability.

Local tolerability measures of the signs and symptoms of skin irritation were considered adverse effects only if the severity of the expected signs and symptoms was such that an interruption of the subject's participation in the study, at his/her request or at the Investigator's discretion, had occurred. Altered dosing regimens (such as every other day dosing) to manage irritation were not considered to be an interruption of the subject's participation in the study.

### EFFICACY ASSESSMENT

### Efficacy Criteria

The primary efficacy criteria were:
- Percent of subjects who are clear or arc almost clear at Week 12, as judged by Evaluator's Global Assessment Score
- Percent of subjects who are clear or almost clear at Week 4, and Week 8, as judged by Evaluator's Global Assessment Score

The secondary efficacy criteria were:
- Change from baseline at Week 4, 8, and 12 in inflammatory lesions counts;
- Change from baseline at Week 4, 8, and 12 in non-inflammatory lesion counts;
- Change from baseline at Week 4, 8, and 12 in total lesions counts;

### Efficacy Measurements

Total lesion counts were performed by trained personnel and included a designation of the number of open comedones, closed comedones, papules, pustules, nodules and cysts. Inflammatory and non-inflammatory counts were determined from the total lesion count using the appropriate designated lesion-types for each category.

Inflammatory lesions are defined as follows:
- Papule - a small, solid elevation less than 5 mm in diameter. Most of the lesion is above the surface of the skin
- Pustule - a small, circumscribed elevation less than 5 mm in diameter that contains yellow-white exudates
- Nodule - a lesion greater than or equal to 5 mm in diameter

Non-inflammatory lesions are defined as follows:
- Open comedones (black head) - a lesion in which the follicle opening is widely dilated with the contents protruding out onto the surface of the skin, with compacted melanin cells giving the plug a black appearance.
- Closed comedone (white head) - a lesion in which the follicle opening is closed. but the sebaccous gland is enlarged by the pressure of the sebum build up, which in turn causes the skin around the follicle to thin and become elevated with a white appearance.

The Evaluator Global Assessment will be judged on a 6-point scale where:
0 = Clear, Normal clear with no evidence of acne
1 = Almost clear, Rare non-inflammatory lesions present, with rare non-inflamed papules; *(papules must be resolving and may be hyperpigmented, though not pinkred)*
2 = Mild, Some non-inflammatory lesions present, with few inflammatory lesions present; *(papules*/*pustutes only; no nodulo-cystic lesions)*
3 = Moderate, Non-inflammatory lesions pre-dominate, with multiple inflammatory lesions evident; *(several to many comedones and papules*/*pustules, and there may or may not be one small nodulo-cystic lesion)*
4 = Severe, Inflammatory lesions are more apparent; *(many comedones and papules*/*pustules, there may or may not be a few nodulo-cystic lesions)*
5 = Very severe, Highly inflammatory lesions predominate; *(variable number of comedones, many papules*/*pustules, nodulo-cystic lesions)*

### SAFETY ASSESSMENT

Safety assessment was conducted for all Subjects at each visit after enrolment in the study.

All clinical medical events, whether observed by the Investigator or reported by the Subject and whether or not thought to be drug-related, was considered adverse events and recorded on the appropriate Adverse Event form (see section 7 for the follow-up of AE).

### Tolerability Assessment

Tolerability was assessed separately by the investigator using the following scales.

**Erythema:** abnormal redness of the skin.
None 0 No erythema
Mild I Slight pinkness present
Moderate 2 Definite redness, easily recognized
Severe 3 Intense redness

**Scaling**: abnormal shedding of the stratum corneum.
None 0 No scaling
Mild 1 Barely perceptible shedding
Moderate 2 Obvious but not profuse shedding
Severe 3 Heavy scale production

**Dryness**: brittle and/or tight sensation
None 0 No dryncss
Mild I Slight but definite roughness
Moderate 2 Moderate roughness
Severe 3 Marked roughness

**Stinging/Burning**: prickling pain sensation immediately after (within 5 minutes of) dosing.
(Requires discussion with subject for evaluation)
None 0 No stinging/burning
Mild I Slight warm, tingling sensation; not really bothersome
Moderate 2 Definite warm, tingling/stinging sensation that is a bother
Severe 3 Hot, tingling/stinging sensation that causes definite bother

### Subject's Satisfaction Questionnaire

At the end of the study, Subjects completed a satisfaction questionnaire regarding the treatment they have been using in this study. All questions were answered numerically on a scale of 1-10 with 10 being the best.
1. Overall, how well did you like using the treatment?
2. Was the treatment irritating?
3. Would you like to continue using the same treatment?
4. Do you believe that the treatment was effective at treating your acne?
5. Overall, how do you feel about your appearance?

### ADVERSE EVENTS

Throughout the course of the study, all adverse events were monitored and reported on an Adverse Event Form without omitting any requested and known information. When adverse events occur, the main concern is the safety of the study Subjects.

### Adverse Events (AEs)

An AE was defined as any unfavorable and unintended sign (e.g., including a clinically relevant abnormal laboratory finding), symptom, or disease temporally associated with the use of a medicinal (investigational) product, whether or not related to the investigational product. Any new sign, symptom or disease, or clinically significant increase in the intensity of an existing sign, symptom or disease, was considered an AE. This included any new signs or symptoms suffered by the subject after accidental or intentional overdose or misuse. Lack of efficacy of the study drug was not considered an AE unless it led to other unfavorable medical occurrences. However, clinically significant worsening of the treated disease was considered an AE.

Pregnancy was not considered an AE but was an important medical event.

Severity of an AE was rated as mild, moderate, or severe. Relationship of an AE to study drug was rated as: related (possibly, probably or definitely related) or unrelated (unlikely or definitely unrelated).

### DEFINITIONS

### Adverse Events (AE)

An adverse event (AE) can be any unfavorable and/or unintended sign (including an abnormal laboratory finding), symptom, or disease temporally associated with the use of a medicinal (investigational) product, whether or not related to the investigational product.

Thus any new sign, symptom or disease, or clinically significant increase in the intensity of an existing sign, symptom or disease, should be considered as an adverse event.

### Notes:

- Clinically significant worsening of the disease/condition being evaluated, which occurs during the study, is considered an adverse event. Lack of clinical endpoint of the study product is not considered an adverse event unless it leads to other unfavorable medical occurrence.
- Any new sign or symptoms suffered by the Subject which appears after accidental or intentional overdose or misuse should also be reported as an adverse event.

Any adverse event, whether or not it is related to the study products, will be reported on the Adverse Event form along with the date of onset, the severity, the relationship with the study product and the outcome.

If the Subject discontinues due to an Adverse Event, the Adverse Event and Exit Forms must be completed.

A few of the most common side effects that may be experience with the use of Differin® Gel, 0.1 % include; redness, dryness, peeling and stinging. Light therapy has no known side effects. The course of these expected events will be assessed and reported on the tolerability assessments. An entry will be made on the Adverse Event Form for all adverse events.

### Serious Adverse Events (SAE)

A serious adverse event is any untoward medical occurrence that at any dose:
- results in death,
- is life-threatening,
- requires inpatient hospitalization or prolongation of existing hospitalization,
- results in persistent or significant disability/incapacity, or
- results in a congenital anomaly/birth defect.

And also:
• Other important medical events that jeopardize the Subject or require intervention to prevent one of the outcomes listed above.

Note: The term "life-threatening" refers to an event in which the Subject was at risk of death at the time of event; it does not refer to an event that hypothetically might have caused death if it was more severe.

Hospitalization solely for the purpose of diagnostic tests, even if related to an adverse event, elective hospitalization for an intervention which was already planned before the inclusion of the Subject in the study, and admission to a day-care facility may not themselves constitute sufficient grounds to be considered as a serious adverse event.

Any pregnancy occurring during clinical trials, where the fetus could have been exposed to the investigational product(s), must be reported in the same manner as a SAE and followed-up until outcome in order to ensure the complete collection of safety data on Galderma Laboratories products.

### Severity

Severity is a clinical determination of the intensity of an adverse event.

The severity assessment for an adverse event is to be completed using the following definitions as a guideline for all adverse events occurring during clinical trials conducted or sponsored by Galderma Laboratories L.P.:
Mild Awareness of sign or symptom, but easily tolerated
Moderate Discomfort, enough to cause interference with usual activity
Severe Incapacitating with inability to work or perform usual activity

### Relationship to Study product

The relationship assessment for an adverse event is to be completed using the following definitions as a guideline for all adverse events occurring during clinical trials conducted or sponsored by Galderma Laboratories L.P.:
Definitely unrelated: Should be reserved for those events which occur prior to study product administration (e.g., washout or single-blind placebo) or for those events which cannot be even remotely related to study participation (e.g., injuries sustained in an automobile accident).
Unlikely: There is no reasonable temporal association between the study product and the event could have been produced by the Subject's clinical state or other modes of therapy administered to the Subject.
Possible: The event may or may not follow a reasonable temporal sequence from study product administration but seems to be the type of reaction that cannot be dismissed as unlikely. The event could have been produced or mimicked by the Subject's clinical state or by other modes of therapy concomitantly administered to the Subject.
Probable: The event follows a reasonable temporal sequence from study product administration, abates upon discontinuation of the product, and cannot be reasonably explained by the known characteristics of the Subject's clinical state.
Definitely related: Should be reserved for those events which have no uncertainty in their relationship to study product administration: this means that a rechallenge was positive.

### PROCEDURES FOR REPORTING ADVERSE EVENTS

At cach visit, the Investigator will question the Subject about adverse events using an open question taking care not to influence the Subject's answer (e.g. "Have you noticed any change in your health since the last visit?").

Directed questioning and examination will then be done when appropriate. All reported adverse events will be documented on the appropriate Case Report Form without omitting any requested and known information.

Every time a concomitant therapy is reported during the study, an Adverse Event Form will be completed if appropriate and the reason for the treatment noted.

### STATISTICAL METHODS

### 8.1. STATISTICAL AND ANALYTICAL PLANS

The main purpose is to demonstrate efficacy, in terms of lesion counts and investigator's global assessment of acne severity.

### Variables to be analyzed

The following variables were analyzed:
• Demographics: Age, gender, skin type, and race.

### Efficacy assessments:

The primary efficacy endpoints were:
- Percent of subjects who are clear or are almost clear at Week 12, as judged by Evaluator's Global Assessment Score
- Percent of subjects who are clear or almost clear at Week 4, and Week 8, as judged by

Evaluator's Global Assessment Score

The secondary efficacy criteria were:
- Change from baseline at Week 4 and 8 and 12 in inflammatory lesions counts;
- Change from baseline at Week 4 and 8 and 12 in non-inflammatory lesion counts;
- Change from baseline at Week 4 and 8 and 12 in total lesions counts;

### Safety assessments:

- Tolerability Assessment
- Incidence of Adverse Event(s)

### Populations Analyzed, Evaluability and Limitation / Evaluation of Bias

The following populations were analyzed:
1. The per-protocol (PP) efficacy population
   This population consists of all enrolled and randomized Subjects, except Subjects considered not evaluable due to major deviations from the protocol. Major deviations were defined after data entry and before unblinding the study treatment, and may include: inclusion/exclusion criteria not respected, non-available efficacy assessment, interfering therapy at inclusion, visit window discrepancy, etc.
2. The intent-to-treat (ITT) efficacy population
   This population consists of the entire population enrolled and randomized (e.g., assigned a subject number). This population was analyzed using the last observation carried forward (LOCF) method to impute missing values.
3. Safety
   Any patient who has received at least one dose of study medication was evaluable for safety.

### Data Presentation and Graphics

All efficacy variables and tolerability assessment variables were summarized by treatment group at each visit and LOCF endpoint. Frequencies were used to summarize categorical variables by treatment group and visit. The continuous variables were summarized by treatment group at each visit using mean (standard deviation), or median (range). The efficacy and tolerability assessment variables were summarized based on both ITT and PP populations.

For the safety variables, all summaries are based on the safety population. Tolerability assessments are summarized by severity score by treatment group at each visit. Overall adverse events are tabulated in frequency tables by body system. Additional summary is provided for adverse events that are considered serious (SAEs), related to the study product, and leading to discontinuation. All AE summary are based on the number of Subjects who experience AE(s). For a given AE, a Subject was counted once even if he or she has experienced multiple episodes for that particular AE.

### Planned Methods of Analysis

The percent of subjects who are clear or almost clear at each of weeks 4, 8, and 12 was reported for each treatment group. A generalized estimating equation (GEE) method for dichotomous data was used to compare the three treatment groups at each of the three time points.

Mean change from baseline in each of inflammatory lesion counts, non-inflammatory lesion counts, and total lesion counts was estimated for each treatment group at each of weeks 4, 8, and 12. For each of the three lesion count variables, a linear mixed effects analysis of variance will be used to compare mean count of each of the three treatment groups at each time point separately.

The distribution of the tolerability assessment scores was described for each treatment group at each time point. A generalized estimating equation approach for ordinal data was used to compare the three treatment groups at each time point.

Since this is not a phase III pivotal trial, no adjustments for multiple comparisons was made. The critical value for each comparison was a = 0.05.

### SAMPLE SIZE DETERMINATION

### Sample size calculation

The current trial is designed as a pilot study to investigate differences in efficacy and safety between the three treatment groups. The sample size of 20 Subjects in each treatment group is not large enough to provide reasonable power to detect statistically significant differences between treatment groups, if such differences do indeed exist. Regardless, differences between treatment groups were summarized and compared in a descriptive and statistical manner. If any significant differences between groups are found, then there will be evidence that the results are robust.

### RESULTS

Assessments were made at baseline, week 4, week 8 and week 12. Total, inflammatory and non-inflammatory lesion counts were captured at each assessment visit. The combination therapy approach yielded the best lesion count reduction of the three treatment arms.

According to the resulsts presented below, the combination of Adapalene 0.1% Gel with Red and/or Blue light is therefore more effective than either treatment Alone for Acne vulgaris.

### EFFICACY RESULTS:

For the Differin only arm, 17 subjects were enrolled, none were excluded from the Intent to Treat population, and 14 were excluded from the Per Protocol population, mostly due to subjects' missing a visit or subjects' visit off schedule by more than 5 days.

For the light only arm, 17 subjects were enrolled, none were excluded from the Intent to Treat population, and 4 were excluded from the Per Protocol population, mostly due to subjects' visit off schedule by more than 5 days.

For the light / Differin arm, 18 subjects were enrolled, none were excluded from the Intent to Treat population, and 4 were excluded from the Per Protocol population, mostly due to subjects' missing a visit or subjects' visit off schedule by more than 5 days.

As a result of the small sample size in the Differin only arm per protocol population, the intent-to-treat population was exclusively used for analysis, where appropriate.

### Primary Efficacy End-point

The primary endpoints were the percent of subjects who clear or are almost clear at Week 12, as judged by Evaluator's Global Assessment Score and the percent of subjects who are clear or almost clear at Week 4, and Week 8, as judged by Evaluator's Global Assessment Score. The combination of light and Diffcrin resulted in a larger percentage of clear or almost clear scores on the Evaluator's Global Assessment at week 12 (22% for the combination vs. 6% for Differin only and 12% for light only). The same assessment is true of week 8, but a much smaller effect is present (11% for the combination vs. 0% for Differin only and 6% for light only). At week 4, there is virtually no difference between treatment arms. Note that no statistical assessment of these percentages was performed due to the small sample size of this pilot study.

### Number and Percent of Subjects Who Are Clear or Almost Clear According to Evaluator's Global Assessment Score (Table I)

**Table I :**

| | | **Differin Only** | **Light Only** | **Light / Differin** |
|---|---|---|---|---|
| **Intent to Treat** | | | | |
| Number of Subjects: | | 17 | 17 | 18 |
| | Baseline | 0 (0%) | 0 (0%) | 0 (0%) |
| | Week 4 | 0 (0%) | 1 (6%) | 1 (6%) |
| | Week 8 | 0 (0%) | 1 (6%) | 2 (11%) |
| | Week 12 | 1 (6%) | 2 (12%) | 4 (22%) |

### Secondary Endpoints

The secondary endpoints were the percent change from baseline at Week 4 and 8 and 12 in inflammatory lesions counts, percent change from baseline at Week 4 and 8 and 12 in non-inflammatory lesion counts, and percent change from baseline at Week 4 and 8 and 12 in total lesions counts.

For each secondary endpoint, statistical hypothesis tests were performed, comparing the light only group's percent change from baseline to the light / Differin group's percent change from baseline. Each p-value was less than 0.001, indicating that the combination of light and Differin reduced the lesion counts more than using just light alone. Note that the Differin only group resulted in smaller or comparable percent differences. As a result, it can be inferred that the combination of light and Differin reduced the lesion counts more than using just Differin alone.

Additionally, generalized estimating equations were used to estimate p-values for a hypothesis test that compared the percent change in lesion count from baseline at each week to 0. For the percent change from baseline in inflammatory lesion analysis, each treatment's change was statistically significant at week 12. However, at week 4, only the light / Differin treatment arm was significant, indicating that lesion counts decrease faster when using the combination of light and Differin. Similar conclusions hold for the analysis of non-inflammatory lesion counts and the analysis of the total lesion counts.

For this analysis, the statistical analyses did not control for race, gender, age, and skin type due to the small size and nature of the study. However, the results of the analyses do not indicate that controlling for the demographic variables was necessary (table II).

**Table II**

| **% Change from Baseline in Inflammatory Lesion Counts** | | | | | |
|---|---|---|---|---|---|
| | | | **Differin Only** | **Light Only** | **Light / Differin** |
| **Intent to Treat** | Number of Subjects: | | 17 | 17 | 18 |
| | Week 4 | Mean (SD) p-value¹ | -10.4 (16.54) 0.126 | -8.8 (25.60) 0.195 | -30.6 (26.73) <0.001 |
| | Week 8 | Mean (SD) p-value¹ | -18.6 (23.02) 0.007 | -20.6 (33.38) 0.003 | -42.9 (28.79) <0.001 |
| | Week 12 | Mean (SD) p-value¹ | -19.1 (36.81) 0.006 | -34.0 (26.09) <0.001 | -45.3 (28.17) <0.001 |
| | | | | | |

| **% Change from Baseline in Non-Inflammatory Lesion Counts** | | | | | |
|---|---|---|---|---|---|
| | | | **Differin Only** | **Light Only** | **Light / Differin** |
| **Intent to Treat** | Number of Subjects: | | 17 | 17 | 18 |
| | Week 4 | Mean | -3.8 (22.03) | -24.0 | -18.9 |
| | | (SD) p-value¹ | 0.583 | (23.24) <0.001 | (40.50) 0.006 |
| | Week 8 | Mean (SD) p-value¹ | 8.5 (17.20) 0.224 | -31.2 (27.10) <0.001 | -35.5 (33.00) <0.001 |
| | Week 12 | Mean (SD) p-value¹ | -19.3 (30.07) 0.007 | -30.8 (28.18) <0.001 | -36.0 (29.28) <0.001 |
| | | | | | |

| **% Change from Baseline in Total Lesion Counts** | | | | | |
|---|---|---|---|---|---|
| | | | **Differin Only** | **Light Only** | **Light / Differin** |
| **Intent to Treat** | Number of Subjects: | | 17 | 17 | 18 |
| | Week 4 | Mean (SD) p-value¹ | -6.4 (15.42) 0.254 | -19.0 (20.36) 0.001 | -23.5 (26.15) <0.001 |
| | Week 8 | Mean (SD) p-value¹ | -12.3 (16.05) 0.030 | -26.9 (24.38) <0.001 | -38.6 (28.23) <0.001 |
| | Week 12 | Mean (SD) p-value¹ | -19.8 (26.73) <0.001 | -31.5 (21.35) <0.001 | -39.7 (24.54) <0.001 |

| | | | | | |
|---|---|---|---|---|---|
| 1. The p-value is from a hypothesis test for comparing the percent change between the week's visit and baseline to 0. | | | | | |

### SAFETY RESULTS:

### Tolerability Evaluation - Erythema,Peeling/Scaling, Dryness, Stinging/Burning

At baseline, the treatment arms had comparable tolerability profiles, as greater than 80% of the subjects responding to each of the tolerability measures as "None." All of the subjects responded to each of the tolerability measures as either "None" or "Mild."

At the end of the study (week 12), at least 80% of the subjects in each of the light only and combination light / Differin treatment arms responded to each of the tolerability measures as "None." In the light only treatment arm, all of the subjects responded to each of the tolerability measures as either "None" or "Mild." In the combination light / Differin treatment arm, 2 subjects reported moderate erythema, 1 subject reported moderate peeling/scaling, I subject reported moderate dryness, and 1 subject reported moderate stinging/burning.

In the Differin only treatment arm, most (at least 50%) subjects responded to each of the tolerability measures as "Nonc." However, 1 subject reported moderate crythema, 1 subject reported moderate dryness, and 1 subject reported moderate stinging/burning at the end of the study (week 12).

### Adverse Events

For the Differin only group, 1 subject out of 12 (8%) reported 2 events, and both events were mild. One adverse event was possibly related to the treatment, and one was probably related to the treatment.

For the light only group, 9 subjects out of 17 (53%) reported 9 events, with 6 events being mild and 8 events being moderate. Of these adverse events, 6 were definitely unrelated to the treatment, 7 were unlikely related to the treatment, and 1 was definitely related to the treatment. This adverse event was claustrophobia caused by the Omnilux procedure.

For the light / Differin group, 8 subjects out of 17 (47%) reported 15 events, with 7 events being mild and 8 events being moderate. Of these adverse events, one was definitely unrelated to the treatment, one was unlikely related to the treatment, one was possibly related to the treatment (acne aggravated), 4 were probably related to the treatment (application site erythema, application site irritation, and dermatitis contact (2)), and 8 were definitely related to the treatment (application site exfoliation, application site dryness (4), application site irritation, sunburn, and skin exfoliation).

### Subject Satisfaction Questionnaire

At the end of treatment, subjects were asked to rate various aspects of the treatment from 1 to 10, with 1 representing the worst and 10 representing the best. The aspects included whether the subject liked the treatment, found the treatment irritating, would like to continue the same treatment, believed the treatment was effective, and was satisfied with his/her appearance. The results are tabulated below. Overall, subjects preferred using light over Diffcrin only (median of 8 for each of light only and light / Differin combination versus a median of 7 for the Differin only treatment), would like to continue using light (median of 9 and 8.5 for the light only and light / Differin combination, respectively, versus a median of 6 for the Differin only treatment), believed the light treatment was effective (median of 7.5 for the light / Differin combination versus a median of 5.5 for the Differin only treatment), and was more satisfied with their appearance after using light (median of 7 for the light / Differin combination versus a median of 5 for the Differin only treatment) (Table III).

**Table III:**

| **Intent to Treat** | | **Differin Only** | **Light Only** | **Light Differin** |
|---|---|---|---|---|
| Number of Subjects with Answer: | | 10 | 16 | 14 |
| (1 = worst; 10 = best) | | | | |
| Q1 - Overall, how well did you like using the treatment? | Mean (SD) | 6.4 (2.46) | 7.7 (2.02) | 8.2 (1.85) |
| | Median (Min-Max) | 7.0 (2-10) | 8.0 (4-10) | 8.0 (3-10) |
| Q2 - Was the treatment irritating? | Mean (SD) | 7.9 | | |
| | Median (Min-Max) | (2.02) | 9.7 (1.01) | 7-3 (2.52) |
| | | 8.0 (3-10) | 10.0 (6-10) | 8.0 (2-10) |
| Q3 - Would you like to continue using the same treatment? | Mean (SD) | 5.5 (3.75) | 8.1 (2.77) | 7.9 (2.37) |
| | Median (Min-Max) | 6.0 (1-10) | 9.0 (1-10) | 8.5 (2-10) |
| Q4 - Do you believe that the treatment was effective at treating your acne? | Mean (SD) | 5.7 (3.02) | 7.4 (1.89) | 7.4 (1.82) |
| | Median (Min-Max) | 5.5 (1-10) | 8.0 (4-10) | 7.5 (4-10) |
| Q5 - Overall, how do you feel about your appearance? | Mean (SD) | 5.7 (2.79) | 7.4 (1.78) | 7.0 (2.51) |
| | Median (Min-Max) | 5.0 (1-10) | 7.0 (4-10) | 7.0 (1-10) |

### Bayesian Hypothesis Test

Due to the small sample size in the per protocol population of the Differin only treatment arm, a Bayesian analysis was performed to estimate the proportion of successes for each treatment arm and then compare each treatment arm. The results of this analysis showed that, at the end of treatment (week 12), there is a 91 % greater chance that the combination of light and Differin will be successful compared to using Differin alone.

### CONCLUSIONS:

The use of light combined with Differin was tolerated at least as well as using Differin alone. At least 80% of the subjects reported either none or mild to each of the tolerability measures. The other subject's worst responses were moderate erythema, moderate scaling/peeling, moderate dryness, or moderate stinging/burning.

Subjects preferred using a light treatment and Differin over using just Differin in their treatment regimen, even though the combination of Omnilux and Differin resulted in 13 more adverse events, with 12 (80%) adverse events probably or definitely related to the treatment.

The primary endpoints compared the three treatment group's percentages of subjects who reported clear or almost clear at the end of the study. The results of the analysis of the primary endpoint are that 22% of the subjects reporting clear or almost clear using the Omnilux system in conjunction with Differin, 12% of the subjects reporting clear or almost clear using only the Omnilux system, and 6% of the subjects reporting clear or almost clear using only Differin.

Lastly, the secondary endpoints compared lesion counts at weeks 4, 8, and 12 to the baseline lesion counts for each treatment arm. The result of the analysis of the secondary endpoint is that the combination of Omnilux and Differin reduce the number of lesions faster than either of the two treatments alone.

This example demonstrates that the combination of Adapalene 0.1 % Gel with Red and/orBlue light is therefore more effective than either treatment Alone for Acne vulgaris.

## Claims

1. A naphtoic acid derivative which is Adapalene at a concentration of 0.1 or 0.3% by weight relative to the total weight of the composition, in association or combination with red and/or blue light exposure, for use in inhibiting or treating acne related diseases.

2. The association or combination for the use according to claim 1, wherein acne related diseases is acne vulgaris.

3. The association or combination for the use according to claim 1 or 2, wherein the treatment with the naphtoic acid derivative and light exposure is carried out at least once per day between every two day to once a week.

4. The association or combination for the use according to claim 1 to 3, wherein the treatment with the naphtoic acid derivative and light exposure is carried out once per day twice a week.

5. The association or combination for the use according to claim 1 to 4, wherein the naphtoic acid derivative treated afflicted skin is exposed to blue light first and red light then.

6. The association or combination for the use according to claim 5, wherein the naphtoic acid derivative treated afflicted skin is exposed to blue light the first week and the red light exposure is carried out the weeks following.

7. The association or combination for the use according to claim 1, wherein the blue light is emitted with length waves comprised between 405 nm and 420 nm, preferably between 410 nm and 420 nm, most preferred at 415 nm.

8. The association or combination for the use according to claim 1, wherein the red light is emitted with length waves comprised between 620 nm and 650 nm, preferably between 630 nm and 640 nm, most preferred at 633 nm.

9. The association or combination for the use according to claim 1 to 8, wherein the treatment with the naphtoic acid derivative and light exposure is carried out for at least 3 weeks and preferably over a period between 4 and 12 weeks.

10. The association or combination for the use according to claim 1 to 9, wherein the treatment with the naphtoic acid derivative and light exposure is carried out on skin, preferably on face, trunk and back.

11. Use of a naphtoic acid derivative which is adapalene at a concentration of 0.1 or 0.3% by weight relative to the total weight of the composition, in the preparation of a topical medicament for administering to a patient in need so as to provide a treatment of acne related disease in association or combination with red and/or blue light.

12. Use according to claim 11, wherein acne related disease is acne vulgaris.

13. Use according to claim 11, wherein the topical medicament is an aqueous gel composition.

## Patentansprüche

1. Naphthoesäurederivat, wobei es sich um Adapalen in einer Konzentration von 0,1 oder 0,3 Gew.% mit Bezug auf das Gesamtgewicht der Zusammensetzung handelt, in Verbindung oder in Kombination mit der Aussetzung an rotes und/oder blaues Licht, zur Verwendung bei der Hemmung oder Behandlung Acne-bezogenen Krankheiten.

2. Verbindung oder Kombination zur Verwendung nach Anspruch 1, wobei die Acne-bezogene Krankheit Acne vulgaris ist.

3. Verbindung oder Kombination zur Verwendung nach Anspruch 1 oder 2, wobei die Behandlung mit dem Naphthoesäurederivat und der Aussetzung an Licht mindestens einmal pro Tag durchgeführt zwischen jedem zweiten Tag und einmal in der Woche durchgeführt wird.

4. Verbindung oder Kombination zur Verwendung nach Anspruch 1 bis 3, wobei die Behandlung mit dem Naphthoesäurederivat und der Aussetzung an Licht einmal pro Tag zweimal in der Woche durchgeführt wird.

5. Verbindung oder Kombination zur Verwendung nach Anspruch 1 bis 4, wobei die mit Naphthoesäurederivat behandelte befallene Haut zuerst an blaues Licht und dann an rotes Licht ausgesetzt wird.

6. Verbindung oder Kombination zur Verwendung nach Anspruch 5, wobei die mit Naphthoesäurederivat behandelte befallene Haut die erste Woche an blaues Licht ausgesetzt wird und die Aussetzung an rotes Licht die darauf folgenden Wochen durchgeführt wird.

7. Verbindung oder Kombination zur Verwendung nach Anspruch 1, wobei das blaue Licht mit Wellenlängen zwischen 405 nm und 420 nm emittiert wird, vorzugsweise zwischen 410 nm und 420 nm, insbesondere mit 415 nm.

8. Verbindung oder Kombination zur Verwendung nach Anspruch 1, wobei das rote Licht mit Wellenlängen zwischen 620 nm und 650 nm emittiert wird, vorzugsweise zwischen 630 nm und 640 nm, insbesondere mit 633 nm.

9. Verbindung oder Kombination zur Verwendung nach Anspruch 1 bis 8, wobei die Behandlung mit dem Naphthoesäurederivat und der Aussetzung an Licht mindestens 3 Wochen lang durchgeführt wird, und vorzugsweise während eines Zeitraums zwischen 4 und 12 Wochen.

10. Verbindung oder Kombination zur Verwendung nach Anspruch 1 bis 9, wobei die Behandlung mit dem Naphthoesäurederivat und der Aussetzung an Licht auf der Haut, vorzugsweise im Gesicht, auf dem Rumpf und dem Rücken durchgeführt wird.

11. Verwendung eines Naphthoesäurederivats, wobei es sich um Adapalen in einer Konzentration von 0,1 oder 0,3 Gew.% mit Bezug auf das Gesamtgewicht der Zusammensetzung handelt, bei der Zubereitung eines topischen Arzneimittels zur Verabreichung eines Patienten, der dieses erfordert, um eine Behandlung einer Acne-bezogenen Krankheit bereitzustellen, in Verbindung oder in Kombination mit rotem und/oder blauem Licht.

12. Verwendung nach Anspruch 11, wobei die Acne-bezogene Krankheit Acne vulgaris ist.

13. Verwendung nach Anspruch 11, wobei das topische Arzneimittel eine wässrige Gelzusammensetzung ist.

## Revendications

1. Dérivé de l'acide naphtoïque qui est l'adapalène à une concentration de 0,1 % ou de 0,3 % en poids par rapport au poids total de la composition, en association ou en combinaison avec une exposition à une lumière rouge et/ou bleue, pour une utilisation dans l'inhibition ou le traitement d'une maladie liée à l'acné.

2. Association ou combinaison pour l'utilisation selon la revendication 1, dans laquelle la maladie liée à l'acné est l'acné vulgaire.

3. Association ou combinaison pour l'utilisation selon la revendication 1 ou 2, dans laquelle le traitement avec le dérivé de l'acide naphtoïque et par exposition à une lumière est réalisé au moins une fois par jour entre tous les deux jours à une fois par semaine.

4. Association ou combinaison pour l'utilisation selon l'une quelconque des revendications 1 à 3, dans laquelle le traitement avec le dérivé de l'acide naphtoïque et par exposition à une lumière est réalisé une fois par jour deux fois par semaine.

5. Association ou combinaison pour l'utilisation selon l'une quelconque des revendications 1 à 4, dans laquelle la peau lésée traitée avec le dérivé de l'acide naphtoïque est exposée d'abord à une lumière bleue puis à une lumière rouge.

6. Association ou combinaison pour l'utilisation selon la revendication 5, dans laquelle la peau lésée traitée avec le dérivé de l'acide naphtoïque est exposée à une lumière bleue la première semaine et l'exposition à la lumière rouge est réalisée les semaines suivantes.

7. Association ou combinaison pour l'utilisation selon la revendication 1, dans laquelle la lumière bleue est émise à des longueurs d'onde comprises entre 405 nm et 420 nm, de préférence entre 410 nm et 420 nm, et de manière préférée entre toutes à 415 nm.

8. Association ou combinaison pour l'utilisation selon la revendication 1, dans laquelle la lumière rouge est émise avec des longueurs d'onde comprises entre 620 nm et 650 nm, de préférence entre 630 nm et 640 nm, et de manière préférée entre toutes à 633 nm.

9. Association ou combinaison pour l'utilisation selon l'une quelconque des revendications 1 à 8, dans laquelle le traitement avec le dérivé de l'acide naphtoïque et par exposition à une lumière est réalisé pendant au moins 3 semaines et, de préférence, sur une période entre 4 et 12 semaines.

10. Association ou combinaison pour l'utilisation selon l'une quelconque des revendications 1 à 9, dans laquelle le traitement avec le dérivé de l'acide naphtoïque et par exposition à une lumière est réalisé sur la peau, de préférence sur le visage, le tronc et le dos.

11. Utilisation d'un dérivé de l'acide naphtoïque qui est l'adapalène à une concentration de 0,1 % ou de 0,3 % en poids par rapport au poids total de la composition, dans la préparation d'un médicament topique destiné à une administration à un patient en ayant besoin afin de fournir un traitement d'une maladie liée à l'acné en association ou en combinaison avec une lumière rouge et/ou bleue.

12. Utilisation selon la revendication 11, dans laquelle la maladie liée à l'acné est l'acné vulgaire.

13. Utilisation selon la revendication 11, dans laquelle le médicament topique est une composition de gel aqueuse.
